# EUROPEAN PATENT APPLICATION

(11) **EP 2 322 148 A2**
(43) Date of publication of application: **18.05.2011**
(21) Application number: 09805172.5
(22) Date of filing: 04.08.2009
(51) Int. Cl.: A61K 9/70

(54) **AROMA THERAPY PATCH**

(30) Priority: 04.08.2008 KR 20080075978
(71) Applicant: Nirapharm Co., Ltd., Seoul 150-800 (KR)
(72) Inventor: JOO, Hyun-Wook, Pyeongtaek-si Gyeonggi-do 450-746 (KR); BAEK, Sun-Min, Seoul 120-110 (KR)
(74) Representative: von Kreisler Selting Werner
(86) International application number: PCT/KR2009/004346
(87) International publication number: WO 2010/016711

(57) **Abstract**

The present invention relates to an aroma therapy patch including a herbal layer containing herbal extract which is formed on one surface of a substrate having a specific shape, and an adhesive layer formed on the other surface of the substrate, wherein at least one surface of the substrate is colored. The aroma therapy patch of the present invention exerts aromatic effects by the actions of herbal extracts as well as chromatherapeutic effects induced by a specific color used therein, which can be used easily and cost-effectively by an ordinary person.

## Description

### FIELD OF THE INVENTION

The present invention relates to an aroma therapy patch exerting aroma therapeutic effects of particular herbal plants as well as chromatherapeutic effects induced by the wavelength of a specific color used therein, which can be easily applicable to meridians or acupuncture points of a human body, and a method for treating or alleviating diseases, or for improving the body function by using the said patch.

### BACKGROUND OF THE INVENTION

The human body is comprised of five viscera and six entrails, and they work together to maintain bodily health and prevent various diseases. Several meridian systems of bioenergy flows(meridians) control the functions of organs and other parts of the body. The points along the meridians, where the bioenergy("qi") that flows through the body tend to accumulate, are called "acupuncture points". When the bioenergy flow through the meridians is blocked or stagnated, specific stimulation of the acupuncture points improves the flow of bioenergy through the body and makes the internal organs function well. The human body has 12 meridians and 360 acupuncture points along the meridians.

Imbalanced functions of the organs and other parts of the human body can be treated by acupuncture or moxa cauterizing treatment which stimulates the acupuncture points to rebalance the said functions. However, the treatment using acupuncture or moxa cautery is carried out only by trained doctors or skilled professionals, and it is difficult for an ordinary person to self-treat, which may cause side effects such as pains and skin burns. Besides, such methods can be time consuming and costly.

It has been recently reported that the use of herbal plants(aromatic plants) is effective in physiological and psychological therapy. Aroma therapy, a term created by combining "aroma" and "therapy", is a form of alternative medicine using the aromatic effects of herbal plants.

Studies on the effects of color, which is form of light, and its effects on the human body had initially been conducted by Newton in 1966. According to Newton, color can be classified into infrared, ultraviolet, and visible rays, the visible rays then can be divided into 7 colors. Also, various studies using X-ray, laser light, and other means supported the Newton's conception: a color having a specific wavelength which could deeply permeate through the skin to facilitate hormone formation and metabolism. Such therapy using color for treating diseases is called "chromatherapy".

A color has a specific wavelength depending on the light source, and chromatherapy can improve the flow of the bioenergy through the body, when the bioenergy flowing through the body is hampered, by applying a specific color having a specific wavelength or irradiating such light to the troubled spots of the body.

Western therapists trained in chromatherapy suggested that: red is associated with the heart; blue with spleen; green with liver; black with kidney; white with lungs; yellow with stomach; and violet with febrile spots, which is in line with the five colors of the five primary substances defined in the oriental chromatherapy.

The meridians of the human body can be up-regulated according to the wavelength of a specific color. For example, lung meridian can be up-regulated by white, stomach meridian by yellow, and heart meridian by red. Also, the 360 acupuncture points can be properly stimulated by treating them with a specific color. For example, *hoeum* can be stimulated with red; *taechung,* with blue; *changmun,* with orange; and *paekoe,* with violet. However, it must be noted that the acupuncture points may be down-regulated when treated with an inappropriate color.

The shape of the patch also determines the effectiveness of the treatment when it is applied to acupuncture points.

The present inventors have found that a particular acupuncture point can be up-regulated when it is applied with a treatment patch having a shape similar to the shape of the organ it is treating. For example, an oval shape is suitable for the acupuncture point related to the tonsil, an isosceles triangle shape is suitable for the acupuncture point related to the stomach, and circular shape is suitable for the acupuncture point related to the heart.

At present, the aroma therapy is effectuated by limited methods such as fumigation, bathing, liquid solutions, and the like, which are time consuming and has limited availability. Therefore, the present inventors have endeavored to develop an aroma therapy patch having a specific color and shape, which is useful in treating diseases, as well as treating or alleviating specific diseases, or improving body functions when using the said patch.

### SUMMARY OF THE INVENTION

Accordingly, the object of the present invention is to provide an aroma therapy patch having a specific shape, which exerts aromatherapeutic effects of particular herbal plants as well as chromatherapeutic effects induced by the color's wavelength.

In accordance with the objective mentioned above, present invention provides an aroma therapy patch comprised of herbal layer containing herbal extract formed on one surface of a substrate; and an adhesive layer formed on the other surface of the substrate, wherein at least one of the substrate surface has color on it.

Another object of the present invention is to provide a method for treating or alleviating disease, or improving the body function by using the said patch.

In accordance with the objective mentioned above, present invention provides a method for treating or alleviating disease, or improving the body functions of a human body, which involves applying the said aroma therapy patch to appropriate acupuncture points of the human body.

### DETAILED DESCRIPTION OF THE INVENTION

The present invention relates to an aroma therapy patch exerting aroma therapeutic effects by the actions of particular herbal plants as well as chromatherapeutic effects induced by the wavelength of a specific color used therein, which can be easily applicable to a meridian or an acupuncture point of a human body.

The aroma therapy patch of the present invention is comprised of a herbal layer containing herbal extract formed on one surface of a substrate; and an adhesive layer formed on the other surface of the substrate, wherein at least one substrate surface has color on it.

In the present invention, the herbal extract is the extract of a herbal plant selected from the group consisting of lavender, rosemary, lemon, jasmine, chamomile, eucalyptus globulus, peppermint, mate, salvia, thyme, yarrow achillea millefolium, ylang-ylang, tea tree, fennel, cinnamon, frankincense, myrrh, and a mixture thereof. And the above should not be intended to limit the scope of the invention.

The aroma therapy patch of the present invention may comprise at least one of the substrate surfaces having color in them. The color is white, yellow, red, blue, orange, violet, black, green, blue-green, red brown, pink, or navy blue, and the aroma therapy patch may comprise the color of all visible rays.

In an embodiment of the present invention, the aroma therapy patch may be of the shape of a circle, oval, triangle, quadrangle, pentagon, hexagon, heart, semi-circle, or star, which is similar to the organ of human body. And the above should not be intended to limit the scope of the invention.

In an embodiment of the present invention, the herbal layer may further comprise a far-infrared emission material, or an anion emission material. The anion-emission material is charcoal, jade, crystal, tourmaline, or barley stone, and the above should not be intended to limit the scope of the invention. In an embodiment of the present invention, the herbal layer may further comprise additional agents such as a shape-retention agent, or a moisture absorbent, which is used in the preparation of the conventional transcutaneous patch.

The aroma therapy patch of the present invention may come in a suitable size to perform its beneficial effects by applying the aroma therapy patch to the acupuncture point of the human body. Preferably, the patch has a maximum diameter or a maximum side length of 0.5 to 20 cm.

The aroma therapy patch of the present invention may be used in treatment or alleviation of a disease selected from the group consisting of laryngopharyngitis, tonsillitis, dyspepsia, stomachache, bloodshot-eyes, fatigue, menstrual irregularity, ovulation pain, muscle ache, a muscle distortion-related disorder, cold, leucorrhea, prostatic hypertrophy, declined energy, growth disorder, angina pectoris, cardiac neurosis, diabetes, hypertension, constipation, and atopic dermatitis; or improvement of the body functions corresponding to an increase of driving distance in golf and improved vision.

The aroma therapy patch of the present invention may be used in purpose of health improvement, prevention of the diseases, beauty and improvement of the body function, as well as treatment or alleviation of the diseases.

The acupuncture points may be up-regulated depending on the source of herbal plants, and thus the herbal plants may be changed depending on the acupuncture points. For example, lavender may be applied to the *paekoe;* rosemary, to the *hoeum;* and lemon, to the *chung-wan.*

The aroma therapy patch of the present invention may control the depth of the acupuncture points. For example, the depth of the acupuncture points may be controlled by controlling the amounts of the herbal plants, changing the herbal plants, or adding the far-infrared emission material or the anion emission material. Specifically, the extract of the herbal plants stimulates sympathetic nerves depending on the amount of the extract. Also, the lavender may be applied to *paekoe* for stimulating the parasympathetic nerves, and the rosemary may be applied to *paekoe* for stimulating the sympathetic nerves. Also, only herbal extracts may be used for stimulating the parasympathetic nerves, and the far-infrared emission material or the anion emission material may be added to the herbal extract for stimulating the sympathetic nerves.

The meridians of human body can be up-regulated according to the wavelength of a specific color. For example, lung meridian can be up-regulated by white; stomach meridian by yellow; and heart meridian by red.

Also, the 360 acupuncture points can be properly stimulated by being treated with a specific color. For example, *hoeum* can be stimulated with red; *taechung,* with blue; *changmun,* with orange; and *paekoe,* with violet. However, it must be noted that the acupuncture points may be down-regulated when treated with inappropriate color.

The particular acupuncture points can be up-regulated when it is attached with a treatment patch having a shape similar to the organ it is treating. For example, an oval shape is suitable for the acupuncture point related to the tonsil, an isosceles triangle shape is suitable for the acupuncture point related to the stomach, and a circle shape is suitable for the acupuncture point related to the heart.

Following is a method for preparing the aroma therapy patch of the present invention described in more detail.

The substrate used in the present invention may be one suitable for applying to the human body, such as paper, polymer film, felt, or fabric.

The substrate may be one having a color in itself or, if need arise, it may have at least one of the colored substrate surface by coating a colored layer or attaching colored film on the substrate surface to produce various colors on one or both sides of the substrate in accordance with any of the conventional procedures.

The herbal layer may be formed by coating a crude or diluted solution of the conventional herbal extracts on one surface of the substrate, and wherein the far-infrared emission material or the anion emission material is further added to the herbal extracts The amount of the far-infrared emission material or the anion emission material may be changed in accordance with the purpose of the treatment, and it may be used in an amount ranging from 1 to 1000 parts by weight, preferably 10 to 100 parts by weight on the bases of 100 parts by weight of the herbal extract.

In the present invention, the herbal layer may be mixed with a viscosity modifier for preventing the effusion of the herbal extracts onto the skin when applied to the skin. The herbal layer may be in the form of liquid, gel, or polymer, which is made up of aqueous or nonaqueous composition. The viscosity modifier may be selected from the group consisting of polyvinyl alcohol, ethyl cellulose, carbomer, hydroxypropyl cellulose, methacrylic acid polymer, methacrylate polymer, and a mixture thereof.

The adhesive layer may be formed by coating an adhesive on the other surface of the substrate in accordance with the conventional method. The adhesive may be selected from the conventional medical adhesives which have no side effects when applied to the human body and can be easily removed from the skin.

The adhesive layer may be in the form of a liquid, a gel or a polymer, which is made up of aqueous or nonaqueous compositions. The adhesive layer may further comprise conventional additional agents such as a penetration enhancer, a thickening agent which is non-toxic and increases its viscosity without blocking the delivery of active ingredients, an excipient, an emulsifier, an adhesive, a preservative, an anti-foaming agent, an antioxidant, a surfactant and the like. The penetration enhancer is selected from the group consisting of higher alcohol including ethanol, N-desylmethylsulfoxide (nDMS), polyethylene glycol monolaurate, dilaurate, ester, and a mixture thereof, and the above should not be limited the scope of the invention.

Also, the present invention provides a method for treating or alleviating disease, or improving the body function of a human body, which comprises applying the aroma therapy patch of the present invention to appropriate acupuncture points of the human body. In the present invention, the disease is selected from the group consisting of laryngopharyngitis, tonsillitis, dyspepsia, stomachache, bloodshot-eyes, fatigue, menstrual irregularity, ovulation pain, muscle ache, a muscle distortion-related disorder, cold, leucorrhea, prostatic hypertrophy, declined energy, growth disorder, angina pectoris, cardiac neurosis, diabetes, hypertension, constipation, and atopic dermatitis; and improving the body function corresponding to the increase of driving distance in golf and improved vision.

In the present invention, the acupuncture points of human body are the international standard acupuncture point location suitable for treating the diseases which is recognized by WHO informal consultation.

In the present invention, the term "alleviate", as used herein, refers to the advancement of a certain disease after applying the aroma therapy patch having a specific shape which exerts aromatherapeutic effects by the actions of particular herbal plants and chromatherapeutic effects induced by the wavelength of a specific color to appropriate acupuncture points of the human body suffering from a disease compared to the condition before the application of the aromatherapeutic patch.

The 360 acupuncture points can be properly stimulated by being treated with a specific color. For example, *hoeum* can be stimulated with red; *taechung,* with blue; *changmun,* with orange; and *paekoe,* with violet. However, it must be noted that the acupuncture points may be down-regulated when treated with inappropriate color.

The appropriate acupuncture points on the meridians for treating or alleviating the disease are: *chonyu* (TE16) or *yepung* (TE17) area for laryngopharyngitis or tonsillitis; *chung-wan* (CV12) area for dyspepsia or stomachache; *chonjung* (CV17) area for insomnia; *taechung* (LR3) area for bloodshot-eyes or fatigue; *samumgyo* (SP6) area for menstrual irregularity; *songmun* (CV5) area for ovulation pain; painful areas of the body or a sore spot for muscle ache or a muscle distortion-related disorder; *yogu* (LR5) area for cold or leucorrhea; *kihae* (CV1) and *hoeum* (CV6) areas for prostatic hypertrophy or declined energy; *hwaryungmun* (ST24) area for growth disorder; *shinmun* (HT7) area for angina pectoris or cardiac neurosis; *umnungchon* (SP9) and *yangji* (TE4) areas for diabetes; *chungjo* (TE3) area for hypertension; and *chigu* (TE6) area for constipation; *kokchi* (LI11), *kyonu* (LI15) and *chukpin* (KI9) areas for atopic dermatitis. And the above should not be intended to limit the scope of the invention.

Also, the appropriate acupuncture point for improving the body function is *kwanwon* (CV4) and *yangnungchon* (GV34) areas for improvement of driving distance in golf; and *hapkok* (LI4) and *puryu* (KI7) areas for improved vision. And the above should not be intended to limit the scope of the invention.

The following Examples are intended to further illustrate the present invention without limiting its scope.

### Preparation of aroma therapy patch

### Example 1

5 mg of a crude tea tree extract was coated on one surface of a blue non-woven fabric substrate having an adhesive layer formed on the other surface of the substrate. The shape of the substrate is circular having a diameter of 2 cm.

### Example 2

10 mg of a solution containing 10% lemon extract was coated on one surface of a yellow non-woven fabric substrate having an adhesive layer formed on the other surface of the substrate. The shape of the substrate is oval, the larger diameter of which is 4 cm.

### Example 3

10 mg of a crude lavender extract was coated on one surface of a violet paper substrate having an adhesive layer formed on the other surface of the substrate. The shape of the substrate is semi-circular having a diameter of 3 cm.

### Example 4

20 mg of a crude chamomile extract was coated on one surface of a blue polymer film substrate having an adhesive layer formed on the other surface of the substrate. The shape of the substrate is circular having a diameter of 2 cm.

### Example 5

10 mg of a crude ylang-ylang extract was coated on one surface of a red brown non-woven fabric substrate having an adhesive layer formed on the other surface of the substrate. The shape of the substrate is triangular, the length of which is 3 cm.

### Example 6

20 mg of a crude fennel extract was coated on one surface of a pink polymer film of the substrate having an adhesive layer formed on the other surface of the substrate. The shape of the substrate is circular having a diameter of 3 cm.

### Example 7

20 mg of a mixture of a crude myrrh extract and a crystal powder as a far-infrared emission material in a 1:0.5 weight ratio was coated on one surface of a blue-green non-woven fabric substrate having an adhesive layer formed on the other surface of the substrate. The shape of the substrate is rectangle, the larger length of which is 2 cm.

### Example 8

20 mg of a mixture of a crude rosemary extract and a charcoal powder as an anion-emission material in a 1:0.3 weight ratio was coated to one surface of a red non-woven fabric substrate having an adhesive layer formed on the other surface of the substrate. The shape of the substrate was triangular, the length of which is 2 cm.

### Test Example

### Test Example 1

A patch prepared in Example 1 was applied to the sternocleidomastoid muscle area of 50 patients suffering from laryngopharyngitis or tonsillitis, and maintained for a few days. The degree of the symptom alleviation was observed, and the results are shown in Table 1.

### Test Example 2

A patch prepared in Example 2 was applied to the *chung-wan* area of 50 patients suffering from dyspepsia or stomachache, and maintained for a few days. The degree of the symptom alleviation was observed, and the results are shown in Table 1.

### Test Example 3

A patch prepared in Example 3 was applied to the *chonjung* area of 50 patients suffering from insomnia, and maintained for a few days. The degree of the symptom alleviation was observed, and the results are shown in Table 1.

### Test Example 4

A patch prepared in Example 4 was applied to the *taechung* area of 50 patients suffering from bloodshot-eyes or fatigue, and maintained for a few days. The degree of the symptom alleviation was observed, and the results are shown in Table 1.

### Test Example 5

A patch prepared in Example 5 was applied to the *samumgyo* area of 50 patients suffering from menstrual irregularity, and maintained for a few days. The degree of the symptom alleviation was observed, and the results are shown in Table 1.

### Test Example 6

A patch prepared in Example 6 was applied to the *songmun* area of 50 patients suffering from ovulation pain, and maintained for a few days. The degree of the symptom alleviation was observed, and the results are shown in Table 1.

### Test Example 7

A patch prepared in Example 7 was applied to the painful area of the body and a sore spot of 50 patients suffering from muscle ache or a muscle-distortion related disorder, and maintained for a few days. The degree of the symptom alleviation was observed, and the results are shown in Table 1.

### Test Example 8

A patch prepared in Example 8 was applied to the *yogu* area of 50 patients suffering from cold or leucorrhea, and maintained for a few days. The degree of the symptom alleviation was observed, and the results are shown in Table 1.

**Table 1**

| | 1 day later | 3 days later | 5 days later |
|---|---|---|---|
| Test Example 1 | ○ | △ | △ |
| Test Example 2 | △ | ○ | ○ |
| Test Example 3 | X | △ | ○ |
| Test Example 4 | ○ | △ | △ |
| Test Example 5 | ○ | △ | X |
| Test Example 6 | ○ | X | X |
| Test Example 7 | ○ | △ | △ |
| Test Example 8 | ○ | ○ | △ |
| o very alleviated; △ alleviated; X no-alleviated | | | |

As shown in Table 1, the aroma therapy patch of the present invention can treat or alleviate the symptoms of the diseases in a little while without side effects or pains. Also, the aroma therapy patch can be used easily and cost effectively.

While the invention has been described with respect to the above specific embodiments, it should be recognized that various modifications and changes may be made to the invention by those skilled in the art which also fall within the scope of the invention as defined by the appended claims.

## Claims

1. An aroma therapy patch comprised of a herbal layer containing herbal extract formed on one surface of a substrate; and an adhesive layer formed on the other surface of the substrate, wherein at least one of the substrate surface has color.

2. The aroma therapy patch of claim 1, wherein the herbal extract is an extract of a herbal plant selected from the group consisting of lavender, rosemary, lemon, jasmine, chamomile, eucalyptus globulus, peppermint, mate, salvia, thyme, yarrow achillea millefolium, ylang-ylang, tea tree and fennel, cinnamon, frankincense, myrrh, and a mixture thereof.

3. The aroma therapy patch of claim 1, wherein the color is white, yellow, red, blue, orange, violet, black, green, blue-green, red brown, pink, or navy blue.

4. The aroma therapy patch of claim 1, wherein the aroma therapy patch is of the shape of a circle, oval, triangle, quadrangle, pentagon, hexagon, heart, semi-circle, or star.

5. The aroma therapy patch of claim 1, wherein the herbal layer further comprises a far-infrared emission material or an anion emission material.

6. The aroma therapy patch of claim 1, wherein the aroma therapy patch has a maximum diameter or a maximum side length of 0.5 to 20 cm.

7. The aroma therapy patch of the any one of claims 1 to 6, wherein the aroma therapy patch is used for treating or alleviating a disease selected from the group consisting of laryngopharyngitis, tonsillitis, dyspepsia, stomachache, bloodshot-eyes, fatigue, menstrual irregularity, ovulation pain, muscle ache, a muscle distortion-related disorder, cold, leucorrhea, prostatic hypertrophy, declined energy, growth disorder, angina pectoris, cardiac neurosis, diabetes, hypertension, constipation, and atopic dermatitis.

8. A method for treating or alleviating disease, or improving the body function, which comprises applying the aroma therapy patch of claim 1 to appropriate acupuncture points of the human body.

9. The method of claim 8, wherein the disease is selected from the group consisting of laryngopharyngitis, tonsillitis, dyspepsia, stomachache, bloodshot-eyes, fatigue, menstrual irregularity, ovulation pain, muscle ache, a muscle-distortion related disorder, cold, leucorrhea, prostatic hypertrophy, declined energy, growth disorder, angina pectoris, cardiac neurosis, diabetes, hypertension, constipation, and atopic dermatitis.

10. The method of claim 8, wherein the disease is laryngopharyngitis or tonsillitis, and the acupuncture point is *chonyu* (TE16) or *yepung* (TE17) area.

11. The method of claim 8, wherein the disease is dyspepsia or stomachache, and the acupuncture point is *chung-wan* (CV12) area.

12. The method of claim 8, wherein the disease is insomnia and the acupuncture point is *chonjung* (CV17) area.

13. The method of claim 8, wherein the disease is bloodshot-eyes or fatigue, and the acupuncture point is *taechung* (LR3) area.

14. The method of claim 8, wherein the disease is menstrual irregularity and the acupuncture point is *samumgyo* (SP6) area.

15. The method of claim 8, wherein the disease is ovulation pain and the acupuncture point is *songmun* (CV5) area.

16. The method of claim 8, wherein the disease is muscle ache or a muscle-distortion related disorder, and the acupuncture points are painful areas of the body and a sore spot.

17. The method of claim 8, wherein the disease is cold or leucorrhea, and the acupuncture point is *yogu* (LR5) area.

18. The method of claim 8, wherein the disease is prostatic hypertrophy or declined energy, and the acupuncture points are *kihae* (CV1) and *hoeum* (CV6) areas.

19. The method of claim 8, wherein the disease is growth disorder and the acupuncture point is *hwaryungmun* (ST24) area.

20. The method of claim 8, wherein the disease is angina pectoris or cardiac neurosis, and the acupuncture point is *shinmun* (HT7) area.

21. The method of claim 8, wherein the disease is diabetes, and the acupuncture points are *umnungchon* (SP9) and *yangji* (TE4) areas.

22. The method of claim 8, wherein the disease is hypertension and the acupuncture point is *chungjo* (TE3) area.

23. The method of claim 8, wherein the disease is constipation and the acupuncture point is *chigu* (TE6) area.

24. The method of claim 8, wherein the disease is atopic dermatitis, and the acupuncture points are *kokchi* (LI11), *kyonu* (LI15), and *chukpin* (KI9) areas.

25. The method of claim 8, wherein the improving the body function corresponds to the increase of driving distance in golf, and the acupuncture points are *kwanwon* (CV4) and *yangnungchon* (GV34) areas.

26. The method of claim 8, wherein the improving the body function is improved vision, and the acupuncture points are *hapkok* (LI4) and *puryu* (KI7) areas.
